# EUROPÄISCHE PATENTANMELDUNG

(11) **EP 0 587 137 A1**
(43) Veröffentlichungstag der Anmeldung: **16.03.1994**
(21) Anmeldenummer: 93114413.3
(22) Anmeldetag: 08.09.1993
(51) Int. Cl.: A61K 31/44, A61K 9/14

(54) **Adsorbat aus einem Hilfsstoffgemisch und einem nichtfesten Wirkstoff zur Herstellung von Arzneizubereitungen**

(30) Priorität: 11.09.1992 DE 4230371
(71) Anmelder: HOECHST AKTIENGESELLSCHAFT, D-65929 Frankfurt am Main (DE)
(72) Erfinder: Hornykiewytsch, Theophil, Dr., D-65929 Frankfurt/Main (DE)

(57) **Zusammenfassung**

Es werden ein Adsorbat enthaltend ein Pyridin-2,4-dicarbonsäure-N,N'-diamid als Wirkstoff, 10-30 % hochdisperses Siliziumdioxid, 50 bis 70 % eines inerten Hilfsstoffs und 0 bis 30 % Stärke und gegebenenfalls weitere Zusatzstoffe und ein Verfahren zur Herstellung dieses Adsorbats beschrieben.

## Beschreibung

Das Pyridin-2,4-dicarbonsäure-N,N'-(3-methoxy-propyl)amid (auch als HOE 277 bekannt) ist, ebenso wie die übrigen in EP 0 409 119 beschriebenen Diamide, eine bei Zimmertemperatur hochviskose, klebrige Substanz, die stark hygroskopisch ist, Pyridin-2,4-dicarbonsäurediamide inhibieren die Prolin- und Lysinhydroxylase und eignen sich als Fibrosuppressiva und Immunsuppressiva (EP 0 409 119).

Es bestand die Aufgabe, HOE 277 zur Applikation am Menschen mit pharmazeutisch üblichen Hilfsstoffen zu Tabletten zu verarbeiten. Das Format der Tabletten sollte so gewählt werden, daß es auch im internationalen Rahmen, z.B. auch in Japan, noch von Patienten akzeptiert wird. Als Maximalabmessungen wurden 15 x 7 mm vorgegeben. Die Dosierung sollte ca. 50-100 mg pro Tablette betragen.

Die genannten Substanzeigenschaften von HOE 277 sind für die Herstellung von Tabletten sehr ungünstig und führen bei Anwendung normaler Rezeptur und Herstellverfahren, wie sie in Handbüchern der pharmazeutischen Technologie angegeben sind, zu einer Reihe von Schwierigkeiten. Unter anderem klebte HOE 277 sehr stark an der Wand der verwendeten Geräte, z.B. der Mischer.

HOE 277 bildete mit den verwendeten Hilfsstoffen eine feste, mechanische schwer abbaubare Kruste an der Wand. Agglomeratbildung in den Wirkstoff-Hilfsstoff-Mischungen bzw. Granulaten führten zu Inhomogenitäten der daraus hergestellten Tabletten. Die aus dem Granulat gepreßten Tablettenkerne wiesen nur eine geringe Härte auf und waren daher zum Filmcoaten wenig geeignet. Das Filmcoaten ist erforderlich, um den bitteren Geschmack des Wirkstoffes zu maskieren und ihn vor direkter Lichteinwirkung zu schützen.

Es ist schon mehrfach beschrieben worden, Wirkstoffe auf hochdisperses SiO₂ aufzuziehen (z.B. EP-B-0 158 120, EP-A-487 335, US-Patentschrift 2 879 161, J. Pharm. Sci. 61, 1430 (1972) und J. Pharm. Sci. 73, 401 (1984)). Dies geschieht in der Regel, um die Löslichkeit von schwer löslichen Wirkstoffen durch Vergrößerung der Oberfläche zu erhöhen. Zwei Verfahren werden dafür beschrieben. Entweder wird dabei zu einer Lösung des Wirkstoffes hochdisperses SiO₂ unter Rühren zugegeben, bis die Lösung von dem Feststoff vollständig aufgesaugt wurde. Das Lösemittel kann dann durch Trocknen entfernt werden. Alternativ wird der Wirkstoff mit dem SiO₂ in einem geeigneten Mischer gemischt und dabei möglichst homogen auf das SiO₂ aufgezogen.

Durch die besonderen Eigenschaften von HOE 277 lassen sich beide Verfahren nicht anwenden. Das Einrühren von SiO₂ in eine Lösung von HOE 277 in Wasser führte zur Bildung einer pastösen Masse, die zäh an den Geräten haftete und sich nicht mehr rühren ließ.

Die Zugabe von HOE 277 in Substanz bzw. in wäßriger Lösung zu vorgelegtem SiO₂ führt sofort zur Bildung von Agglomeraten bzw. von Krusten an Mischwerkzeug und Behälterwand.

Wir haben nun gefunden, daß diese Schwierigkeiten überwunden werden können, wenn eine Vermischung aus 10-30 % hochdispersem SiO₂, 50-70 % z.B. Lactose und 0-30 % z.B. Maisstärke vorgelegt wird und die Wirkstofflösung langsam zugegeben wird.

Die Erfindung betrifft daher ein Adsorbat enthaltend ein Pyridin-2,4-dicarbonsäure-N,N'-diamid als Wirkstoff, 10-30 % hochdisperses Siliziumdioxid, 50 bis 70 % eines inerten Hilfsstoffs und 0 bis 30 % Stärke.

Das Adsorbat eignet sich zur Herstellung von Arzneizubereitungsformen.

Die Erfindung betrifft ferner ein Verfahren zur Herstellung eines Adsorbats, dadurch gekennzeichnet, daß man ein Gemisch aus 10-30 % hochdispersem Siliziumdioxid, 50-70 % inertem Hilfsstoff und 0 bis 30 % Stärke vorlegt und zu diesem Gemisch eine wäßrige Lösung eines Pyridin-2,4-dicarbonsäure-N,N'-diamids gibt und das erhaltene homogene Adsorbat gegebenenfalls in eine geeignete Darreichungsform überführt.

Die Prozentangaben sind Gewichtsprozente.
Der Wirkstoff ist vorzugsweise HOE 277.

Das hochdisperse SiO₂ hat insbesondere eine Oberfläche zwischen 150-400 m²/g und eine mittlere Größe der Primärteilchen von 6 bis 16 Nanometer.
Pharmazeutisch geeignete inerte Hilfsstoffe sind z.B. Cellulose und modifizierte Cellulose-Derivate, Zucker, Zuckeralkohole, Ca-phosphate und -carbonat, insbesondere Lactose.

Als Stärke eignet sich z.B. Maisstärke, modifizierte Maisstärke, Reis-, Kartoffel- und Weizenstärke. Der SiO₂-Anteil sollte wegen des Volumens und der sonstigen technologisch kritischen Eigenschaften des SiO₂ möglichst gering gehalten werden; ein Verhältnis 1:1 mit dem Wirkstoff HOE 277 erwies sich als ausreichend. Das homogene Adsorbat enthält vorzugsweise 1-30 % Wirkstoff.

Durch schrittweise Zugabe einer konzentrierten Wirkstoff-Lösung (8 mg HOE 277 + 2 mg H₂O) läßt sich ein homogenes Adsorbat herstellen. Diese läßt sich in Hartgelatinekapseln abfüllen oder weiter zu Tabletten bzw. Filmtabletten verarbeiten.

Bei der Herstellung des erfindungsgemäßen Adsorbats werden die üblicherweise unangenehmen Eigenschaften der Wirkstoffe positiv genutzt, d.h. der klebrige, viskose Wirkstoff hat in konzentrierter, wäßriger Lösung die Funktion eines Bindemittels.

Es erweist sich als vorteilhaft für die Granulat- und die Tabletteneigenschaften bei der Überführung in eine Darreichungsform gegebenenfalls einen eigentlichen Binder, z.B. Polyvinylpyrrolidon, modifizierte bzw. pregelatinisierte Stärke, Cellulose-Derivate, z.B. Methyl-, Hydroxypropyl-, Carboxymethyl-Cellulose, Polyethylenglykole, dem homogenen Adsorbat trocken zuzumischen und dann durch Zugabe der noch fehlenden Wassermenge das endgültige Granulat herzustellen. Durch das beschriebene Verfahren kann ein Trockenvorgang eingespart werden. Dies ist aus Gründen der Zeit- und Energieeinsparung vorteilhaft.

Um Tablettenkerne mit ausreichender Härte und schnellem Zerfall herzustellen, ist es notwendig, das Feuchtgranulat vor dem Verpressen scharf auf ca. 10-20 % rel. Feuchte zu trocknen. In der äußeren Phase beim Bestäuben kann durch Zugabe von ca. 5 % mikrokristall. Cellulose die Tablettenhärte deutlich gesteigert werden. Eine weitere Zugabe (mehr als 10 %) führt zu keiner weiteren Verbesserung.

Wegen des bitteren Geschmacks des Wirkstoffs erhalten die aus dem Adsorbat, gegebenenfalls nach Granulation, hergestellten Tabletten üblicherweise einen Lackfilm. Der Lackfilm besteht z.B. aus einem handelsüblichen Filmbildner, einem Weichmacher, einem Pigment und Deckstoff.

Nach dem Filmlackieren wird noch einmal eine ungewöhnliche Erhöhung der Härte um ca. 50 % beobachtet, die ohne negative Auswirkung auf die Zerfallszeit bleibt.

Von erfindungsgemäß hergestellten Tablettenkernen enthaltend HOE 277 wurde die Härte mit einem Erweka-Härtemeßgerät bestimmt. Ferner wurde die Härte der Filmtabletten entsprechend den Beispielen 1 und 2 bestimmt. Die Mittelwerte aus 10 Bestimmungen sind nachfolgend aufgeführt.

| Dosierung | 40 mg | 80 mg |
|---|---|---|
| Härte | 39 N | 52 N |
| Härte Filmtabletten | 61 N | 71 N |

### Beispiel 1: HOE 277 Filmtabletten zu 40 mg

### Zusammensetzung einer Dosierungseinheit

| Substanz | | Menge in der Einzeldosis [mg] |
|---|---|---|
| 1. | HOE 277 | 40.000 |
| 2. | Hochdisperses Siliciumdioxid | 40.000 |
| 3. | Lactose | 80.000 |
| 4. | Maisstärke | 30.000 |
| 5. | Polyvinylpyrrolidon | 10.000 |
| 6. | Natriumstärkeglykolat | 8.000 |
| 7. | Mikrokristalline Cellulose | 10.000 |
| 8. | Magnesiumstearat | 2.000 |
| | Filmdecke | 5.000 |
| | | 225.000 |

Gereinigtes Wasser dient als Granulationshilfsmittel, sowie als Lösemittel bzw. Suspendiermedium für den Wirkstoff und die Bestandteile der Filmdecke und ist kein Bestandteil der fertigen Zubereitung.
2., 3. und 4. werden gemischt. Diesem Gemisch wird schrittweise unterRühren eine konzentrierte, wäßrige Lösung von 1. zugegeben. Das Adsorbat wird trocken mit 5., 6., 7. und 8. vermischt und nach Zusatz von Wasser feucht granuliert und zu Tablettenkernen verpreßt. Diese werden anschließend mit einer Filmdecke gecoatet.

### Beispiel 2: HOE 277 Filmtabletten zu 80 mg

### Zusammensetzung einer Dosierungseinheit

| Substanz | | Menge in der Einzeldosis [mg] |
|---|---|---|
| 1. | HOE 277 | 80.000 |
| 2. | Hochdisperses Siliciumdioxid | 80.000 |
| 3. | Lactose | 160.000 |
| 4. | Maisstärke | 60.000 |
| 5. | Polvvinylpyrrolidon | 20.000 |
| 6. | Natriumstärkeglykolat | 16.000 |
| 7. | Mikrokristalline Cellulose | 20.000 |
| 8. | Magnesiumstearat | 4.000 |
| | Filmdecke | 10.000 |
| | | 450.000 |

Gereinigtes Wasser dient als Granulationshilfsmittel, sowie als Lösemittel bzw. Suspendiermedium für den Wirkstoff und die Bestandteile der Filmdecke und ist kein Bestandteil der fertigen Zubereitung.
Die Herstellung erfolgt wie in Beispiel 1 beschrieben.

### Beispiel 3: HOE 277 Filmtabletten zu 40 mg

### Zusammensetzung einer Dosierungseinheit

| Substanz | | Menge in der Einzeldosis [mg] |
|---|---|---|
| 1. | HOE 277 | 40.000 |
| 2. | Hochdisperses Siliciumdioxid | 40.000 |
| 3. | Lactose | 220.000 |
| 4. | Maisstärke | 70.000 |
| 5. | Polyvinylpyrrolidon | 20.000 |
| 6. | Natriumstärkeglykolat | 16.000 |
| 7. | Mikrokristalline Cellulose | 20.000 |
| 8. | Magnesiumstearat | 4.000 |
| | Filmdecke | 10.000 |
| | | 440.000 |

Gereinigtes Wasser dient als Granulationshilfsmittel, sowie als Lösemittel bzw. Suspendiermedium für den Wirkstoff und die Bestandteile der Filmdecke und ist kein Bestandteil der fertigen Zubereitung.
Die Herstellung erfolgt analog Beispiel 1.
Die Filmtabletten gemäß Beispielen 1 bis 3 sind stabil.

## Patentansprüche

1. Adsorbat enthaltend ein Pyridin-2,4-dicarbonsäure-N,N'-diamid als Wirkstoff, 10-30 % hochdisperses Siliziumdioxid, 50 bis 70 % eines inerten Hilfsstoffs und 0 bis 30 % Stärke und gegebenenfalls weitere Zusatzstoffe.

2. Adsorbat enthaltend Pyridin-2,4-dicarbonsäure-N,N'-(3-methoxy-propyl)amid als Wirkstoff, 10-30 % hochdisperses Siliziumdioxid, 50 bis 70 % Lactose und 0 bis 30 % Maisstärke.

3. Verfahren zur Herstellung eines Adsorbats, dadurch gekennzeichnet, daß man ein Gemisch aus 10-30 % hochdispersem Siliziumdioxid, 50-70 % inertem Hilfsstoff und 0 bis 30 % Stärke vorlegt und zu diesem Gemisch eine wäßrige Lösung eines Pyridin-2,4-dicarbonsäure-N,N'-diamids gibt und das erhaltene homogene Adsorbat gegebenenfalls in eine geeignete Darreichungsform überführt.

4. Verfahren gemäß Anspruch 3, dadurch gekennzeichnet, daß man den eigentlichen Binder dem homogenen Adsorbat zumischt, dann das Gemisch feucht granuliert und das Granulat trocknet.
